(19) Europäisches Patentamt
European Patent Office
Office européen des brevets

(11) **EP 4 201 315 A1**

(12) **EUROPEAN PATENT APPLICATION**

(43) Date of publication:
**28.06.2023 Bulletin 2023/26**

(21) Application number: **22152851.6**

(22) Date of filing: **24.01.2022**

(51) International Patent Classification (IPC):
**A61B 5/0205** (2006.01)     **A61B 5/00** (2006.01)

(52) Cooperative Patent Classification (CPC):
**A61B 5/0205; A61B 5/7275**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **23.12.2021  US 202163293203 P**

(71) Applicant: **Koninklijke Philips N.V.**
**5656 AG Eindhoven (NL)**

(72) Inventors:
• **SOKORELI, Ioanna**
  **Eindhoven (NL)**
• **DEN TEULING, Nicolaas Gregorius Petrus**
  **Eindhoven (NL)**
• **GRASSI, Angela**
  **Eindhoven (NL)**
• **ZHAO, Claire Yunzhu**
  **Eindhoven (NL)**

(74) Representative: **Philips Intellectual Property & Standards**
**High Tech Campus 52**
**5656 AG Eindhoven (NL)**

(54) **METHOD AND SYSTEM FOR SCREENING CARDIAC CONDITIONS OF A PATIENT**

(57)     The present invention relates to a method for screening cardiac conditions of a patient, the method comprising the following steps: calculating (S1) a cardiac risk value based on therapy sensor data by means of a first level of a multi-level procedure, wherein the therapy sensor data is provided from a first data source as sensor data during a therapy of the patient; and

refining (S2) the calculated cardiac risk value based on survey data and/or device data by means of a second level of a multi-level procedure to provide a refined cardiac risk value, wherein the survey data and/or the device data is provided from a second data source by incremental data gathering.

Fig. 1

**Description**

FIELD OF THE INVENTION

The present invention relates to patient screening and, in particular, the present invention relates to a computer-implemented method for screening cardiac conditions of a patient, a medical system, and a computer program element.

BACKGROUND OF THE INVENTION

Heart failure, HF, or other cardiac conditions are highly prevalent in Sleep Disorder Breathing, SDB, population and are often undiagnosed. The number of hospitalizations and the mortality rate increase in cardiac failure patients with associated SDB. Most patients with HF and SDB often complain about similar symptoms which makes it challenging to diagnose both.

Sleep disorder breathing, SDB, is a condition characterized by interrupted breathing during sleep, for longer than 10 seconds at least 5 times per hour, on average, throughout the sleep period. A lot of patients with SDB have multiple comorbidities. These comorbidities have a significant impact on healthcare use and mortality in patients with SDB. The comorbidity burden progressively increases with SDB severity.

In particular, there is a strong association between SDB and cardiovascular diseases. HF or other cardiac conditions are highly prevalent in SDB population; however, they are often undiagnosed. The number of hospitalizations and the mortality rate increase in cardiac failure patients with associated SDB. SDB causes myocardial as well as arterial damage. Untreated SDB might therefore promote the progression of cardiac disease, resulting in heart failure and increased mortality in patients with heart failure. SDB is strongly related and causally contributes to hypertension, HTN, the most common risk factor for ventricular hypertrophy and HF.

Most patients with HF and SDB often complain about similar symptoms which makes it challenging to diagnose both. Symptoms often common in both conditions are fatigue, daytime somnolence, shortness of breath, edema, and nocturnal dyspnea. Due to these overlapping symptoms cardiac conditions might get overlooked in diagnosed SDB population.

Screening all SDB patients for cardiac conditions would be extremely time consuming and would put a huge burden to healthcare providers and healthcare system.

SUMMARY OF THE INVENTION

There may, therefore, be a need for improved means for a tiered screening approach to detect HF or cardiac patients within SDB population and to lower the burden of screening all SDB patients for cardiac conditions by use of pulmonary arterial pressure, PAP, data for initial risk assessment. The object of the present invention is solved by the subject matter of the independent claims, wherein further embodiments are incorporated in the dependent claims.

According to a first aspect, there is provided a method for screening cardiac conditions of a patient. The method comprises.

calculating a cardiac risk value based on therapy sensor data by means of a first level of a multi-level procedure, wherein the therapy sensor data is provided from a first data source as sensor data during a therapy of the patient; and
refining the calculated cardiac risk value based on survey data and/or device data by means of a second level of a multi-level procedure to provide a refined cardiac risk value, wherein the survey data and/or the device data is provided from a second data source by incremental data gathering.

In this way, a method of detecting heart failure or other cardiac conditions is provided in SDB patients using PAP or alternative therapy data in combination with other available sensors.

The present invention advantageously allows an early cardiac condition detection in terms of a benefit for the patient for early and unobtrusive detection of underlying cardiac conditions in SDB patients and consequently early management of the condition.

By managing their condition at an early stage, the patient could eliminate the burden of the condition on their quality of life. The present invention advantageously allows that the patient therefore can learn early on how to manage health behaviors and risk factors and improve their lifestyle. Cardiac conditions remain the main cause of morbidity and mortality and consequently, early diagnosis is of utmost importance.

The present invention advantageously allows lowering the burden of the healthcare system, early detection of cardiac conditions also means lowering the burden to the healthcare system. Health care expenditures related to cardiac conditions are overwhelming. For example, congestive heart failure affects many people and consumes much value in health-care expenditures annually.

The present invention advantageously allows lowering the burden of screening all SDB patients in terms of lowering the burden of screening all SDB patients for cardiac conditions by following a 2-fold assessment. This approach benefits the healthcare provider by allowing to better spend their time and prioritize the patients that need their help without having to spend their time to screen all SDB patients, including those of the lowest risk of developing or having a cardiac condition.

The present invention advantageously allows a tiered screening approach to detect HF or other cardiac conditions within SDB population and lower the burden of screening all SDB patients for cardiac conditions by use of PAP data for initial risk assessment. The present invention advantageously introduces an incremental approach where an initial risk assessment is performed based on therapy data. For the identified high-risk patient, a second assessment is performed taking into account additional device and patient reported data, aiming to increase the certainty of the risk assessment.

Detecting heart failure or cardiac patients among SDB patients using PAP or alternative therapy data in combination with other available sensors can help to lower the burden of screening all SDB patients for cardiac conditions and also to early detect cardiac conditions and manage them on an early stage.

Preferably, the method may be computer-implemented. Further preferably, a medical system may be operated and/or a medical procedure carried out by utilizing the medical system may be performed in an autonomous operating mode. This may be a semi-autonomous operating mode, in which one or more, but not all, procedure steps are supported by human intervention, e.g. by medical staff or technicians, and one or more, but not all, procedure steps are performed in an automatic manner by the medical system, or which may be a fully-autonomous operating mode, in which at least the actual medical procedure. Optionally, the fully-autonomous operating mode may also concern one or more steps of the procedure in general.

According to an exemplary embodiment of the present invention, the therapy sensor data comprises sensor data on ventilation pressure, ventilation volume, ventilation airflow rate, or oxygen partial pressure provided during respiratory ventilation as the therapy of the patient.

According to an exemplary embodiment of the present invention, the therapy sensor data comprises sensor data on a breath rate of the patient or respiration characteristics of the patient, the sensor data provided by at least one microphone recording the sound of breathing of the patient.

According to an exemplary embodiment of the present invention, the survey data comprises data of a push survey provided by a survey of the patient.

According to an exemplary embodiment of the present invention, the survey of the patent is conducted using an application program on a mobile electronic equipment.

According to an exemplary embodiment of the present invention, the device data comprises data blood pressure and body weight.

According to an exemplary embodiment of the present invention, the device data is provided using an application program on a mobile electronic equipment.

According to an exemplary embodiment of the present invention, the method further comprises the steps of further refining the refined cardiac risk value by additional data of the first data source or the second data source or a third data source and by means of a n-th level of a multi-level procedure; wherein n is greater than 2.

According to a second aspect, there is provided a medical screening system for screening cardiac conditions of a patient, the medical screening system comprises a first data source, a second data source and at least one processor.

The at least one processor is configured to calculate a cardiac risk value based on therapy sensor data using a first level of a two-level procedure, wherein the therapy sensor data is provided from the first data source as sensor data during a therapy of the patient.

The at least one processor is configured to refine the calculated cardiac risk value based on survey data and/or device data using a second level of a two-level procedure to provide a refined cardiac risk value, wherein the survey data and/or the device data is provided from the second data source by incremental data gathering.

According to an exemplary embodiment of the present invention, the first data source is a sensor configured to measure sensor data on ventilation pressure, ventilation volume, ventilation airflow rate, or oxygen partial pressure provided during respiratory ventilation as the therapy of the patient. According to an exemplary embodiment of the present invention, the ventilation airflow rate may advantageously be defined as a more granular measurement when compared to others, for instance volume as amount of airflow over time.

According to an exemplary embodiment of the present invention, the first data source comprises a microphone configured to record the sound of breathing of the patient and the first data source is configured to determine a breath rate of the patient or respiration characteristics of the patient based on the recorded sound of the breathing of the patient.

According to an exemplary embodiment of the present invention, the second data source is implemented on a mobile electronic equipment and the second data source is configured to provide the survey data comprising data of a push survey provided by a survey of the patient, wherein the survey of the patent is conducted using an application program on a mobile electronic equipment.

According to a third aspect, there is provided a computer program element, which when executed by a processor is configured to carry out the method of the first aspect, and/or to control a system according to the second aspect.

According to a fourth aspect, there is provided a computer-readable storage or transmission medium, which has stored or which carries the computer program element according to the third aspect.

It is noted that the above embodiments may be combined with each other irrespective of the aspect involved. Accordingly, the method may be combined with structural features of the system of the other aspects and, likewise, the system may be combined with features described above with regard to the method.

These and other aspects of the present invention will become apparent from and elucidated with reference to the embodiments described hereinafter.

BRIEF DESCRIPTION OF THE DRAWINGS

Exemplary embodiments of the invention will be described in the following drawings.

Fig 1 shows in a schematic block diagram a medical screening system for screening cardiac conditions of a patient according to an exemplary embodiment of the present invention.
Fig. 2 shows a flow chart of a method for screening cardiac conditions of a patient according to an exemplary embodiment of the present invention.
Fig. 3 shows a flow chart of a two-level algorithm or a two-level procedure for screening cardiac conditions of a patient according to an exemplary embodiment of the present invention.

DETAILED DESCRIPTION OF EMBODIMENTS

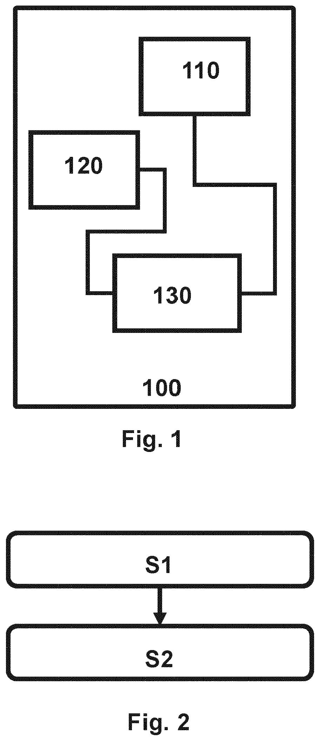

Fig. 1 shows in a schematic block diagram a medical screening system 100 according to an exemplary embodiment of the present invention.

The medical screening system 100 comprises a first data source 110, a second data source 120 and at least one processor 130.

The at least one processor 130 is configured to calculate a cardiac risk value based on therapy sensor data using a first level of a two-level procedure, wherein the therapy sensor data is provided from the first data source as sensor data during a therapy of the patient.

The at least one processor 130 is configured to refine the calculated cardiac risk value based on survey data and/or device data using a second level of a two-level procedure to provide a refined cardiac risk value, wherein the survey data and/or the device data is provided from the second data source by incremental data gathering.

The first data source 110 is configured to provide the therapy sensor data as sensor data during a therapy of the patient and during a first level of a two level procedure.

The second data source 120 is configured to provide the survey data and/or the device data by incremental data gathering and during a second level of the two-level procedure.

Fig. 2 shows in a flow chart of a method for screening cardiac conditions of a patient according to an exemplary embodiment of the present invention.

In a first step, calculating S1 a cardiac risk value based on therapy sensor data by means of a first level of a multi-level procedure is performed, wherein the therapy sensor data is provided from a first data source as sensor data during a therapy of the patient.

In a second step, refining S2 the calculated cardiac risk value based on survey data and/or device data by means of a second level of a multi-level procedure to provide a refined cardiac risk value is conducted, wherein the survey data and/or the device data is provided from a second data source by incremental data gathering.

According to an exemplary embodiment of the invention, the method for screening cardiac conditions of a patient comprises a 2-level scoring algorithm in terms of a 2- or multi-level procedure to calculate a score that indicates a risk of the patient having HF or other cardiac conditions.

According to an exemplary embodiment of the invention, throughout the first assessment phase, a risk is estimated based on therapy data or other sensor data already available to the patient. At the second phase, the assessment is performed by taking into account additional data collected from the patient (either device or self-reported data).

According to an exemplary embodiment of the invention, the algorithm can be implemented as part of the solution already available to the patient, therapy device or mobile application, mobile and internet application.

The present invention provides the advantages of identify patients at risk in an unobtrusive way.

According to an exemplary embodiment of the present invention, the present invention provides the advantage of allowing for later re-evaluation if risk is not assessed as high.

According to an exemplary embodiment of the present invention, the present invention provides the advantage of an increased certainty of the assessment by collecting additional data.

According to an exemplary embodiment of the present invention, the present invention provides the advantage of incremental approach to allow users activate or deactivate elements, or data points, of their interest or elements that might or might not be available to them.

According to an exemplary embodiment of the present invention, the present invention provides the advantages of low cost in its execution.

According to an exemplary embodiment of the present invention, the present invention allows in advantageous manner for integration of any additional available device, for example an activity tracker, or any smart phone.

According to an exemplary embodiment of the present invention, the present invention allows in advantageous manner avoiding any burden to the patient as it requires very limited self-reported information.

According to an exemplary embodiment of the present invention, the present invention provides the advantage of an increase in specificity (and hence precision) of follow-up care provided to the patient.

According to an exemplary embodiment of the present invention, the present invention provides the advantage of a contribution to the overall improvement in early detection of a clinical condition of the patient.

According to an exemplary embodiment of the invention, the method for screening cardiac conditions of a patient comprises an incremental approach:

For the first level of the 2-level scoring algorithm in terms of a 2-level procedure or multi-level procedure, there may be provided an assessment based on PAP data or alternative therapies, e.g. positional therapy.

According to an exemplary embodiment of the invention, the first level of a multi-level procedure may comprise a signal or an alert signal for possible cardiac conditions based on PAP data and partial oxygen pressure or peripheral oxygen saturation, SpO2.

According to an exemplary embodiment of the invention, the data tracking as provided by any data source, i.e. the first or the second data source may comprise change respiration, breath rate, (respiration characteristics of patient), (sound of breathing from home audio devices), etc.

According to an exemplary embodiment of the invention, the data tracking include peripheral oxygen saturation, SpO2 data.

According to an exemplary embodiment of the invention, the second level of a multi-level procedure may comprise incremental data gathering. For part of population with a flag of high risk of cardiac condition

According to an exemplary embodiment of the invention, the second level of a multi-level procedure may comprise a push survey, for instance, a New York Heart Association NYHA approved data survey or patient reported data, to therapy users with symptoms through PAP device of mobile app, such as the Philips DreamMapper application.

According to an exemplary embodiment of the invention, the second level of a multi-level procedure may comprise Additional input from connected devices tracking weight and blood pressure.

According to an exemplary embodiment of the invention, the following modules that are used to calculate a final score estimating a risk of the patient having a cardiac condition.

According to an exemplary embodiment of the invention, as data input modules a first and a second data sources are used. For the first level, the first data source may be used. The data for the first phase assessment can contain data from the therapy device (PAP or positional therapy), peripheral oxygen saturation, SpO2 sensor data, data from wearable device and smartphone data.

According to an exemplary embodiment of the invention, the second level of a multi-level procedure may comprise that additional device data is required and additional patient reported survey data.

According to an exemplary embodiment of the invention, the first assessment data input may comprise therapy data: PAP or positional therapy data about flow and pressure, RR, heart rate, inter-beat intervals, IBIs, apneas, hypopneas, and Cheyne Stokes respiration.

According to an exemplary embodiment of the invention, the first assessment data input may comprise peripheral oxygen saturation, SpO2, sensor data: providing an oxygen saturation and hypoxic burden measurements

According to an exemplary embodiment of the invention, the first assessment data input may comprise wearable device data: any wearable device already available to the patient can be used as additional source of data to increase the risk prediction accuracy. Example of typically available data via such devices is accelerometer data or heart rate

According to an exemplary embodiment of the invention, the first assessment data input may comprise smartphone data: e.g. heart rate HR, sound of breathing, etc.

According to an exemplary embodiment of the invention, the first level of a multi-level procedure may comprise using an algorithmic module for the first phase risk assessment implementing a logistic regression model predicting risk of heart failure, HF, or cardiac condition based on the data inputs mentioned before.

According to an exemplary embodiment of the invention, the first level of a multi-level procedure may comprise using an output of this model is a risk indication: high, medium and low. Based on this indication a follow up step is defined according to one of the exemplary embodiments as follows:

According to an exemplary embodiment of the invention, if patient risk is high then the second assessment phase is performed to increase the risk estimation accuracy.

According to an exemplary embodiment of the invention, if the risk is medium then the assessment is repeated in a follow up time defined by the healthcare provider, for example this may extend only a few weeks, months, for example two weeks.

According to an exemplary embodiment of the invention, if the risk is estimated as low then no direct actions are required. The user can decide if assessment should be repeated in a follow up time, or if patient's conditions change.

According to an exemplary embodiment of the invention, the model output may comprise: Outcome of the logistic regression model P as a probability of having a cardiac condition. Thresholds defined by the user can be applied to identify the patient with the highest risk.

According to an exemplary embodiment of the invention, the model may be defined as follows:

$$P = \frac{e^{(\beta + \alpha_1 x_1 + \cdots \alpha_n x_n)}}{1 + e^{(\beta + \alpha_1 x_1 + \cdots \alpha_n x_n)}}$$

According to an exemplary embodiment of the invention, the intercept $\beta$ and coefficients $\alpha_n$ can be adjusted to avoid over- and underestimation. For that adjustment, we need to fit the logistic model on real data which becomes available after a pilot test of the algorithm for an SDB cohort.

According to an exemplary embodiment of the invention, the second assessment data input in terms of the survey data and/or device data by means of a second level of a multi-level procedure comprises additional device data: e.g. weight measurement, blood pressure measurement.

According to an exemplary embodiment of the invention, the second assessment data input in terms of the survey data and/or device data by means of a second level of a multi-level procedure comprises self-reported data: survey data is collected via PAP machine or a mobile application.

According to an exemplary embodiment of the invention, when performing method using the Philips DreamMapper application where the patient is asked to report certain symptoms for example data on breathlessness, fatigue, oedema (known as fluid retention, dropsy, hydropsy and swelling, is the build-up of fluid in the body's tissue), and answer a question needed to estimate the NYHA class, for example an indication of HF severity, the survey data is collected via PAP machine or a mobile application.

According to an exemplary embodiment of the invention, the second assessment data input in terms of the survey data and/or device data by means of a second level of a multi-level procedure comprises data on push some home test suggestion for patients to evaluate themselves to further improve the accuracy of the second assessment.

According to an exemplary embodiment of the invention, the second assessment data input in terms of the survey data and/or device data by means of a second level of a multi-level procedure comprises data on sudden weight gain, a walk test, suggest to perform annual physical exam and obtain blood tests (e.g. sodium, potassium, albumin, creatinine, LDL, etc.)

According to an exemplary embodiment of the invention, the algorithmic module for the second phase risk assessment based on the survey data and/or device data by means of a second level of a multi-level procedure comprises a second risk model based on logistic regression is used to estimate the risk of HF or other cardiac condition.

According to an exemplary embodiment of the invention, the Input features for this model are all data points collected in both first and second phase assessment. Output of the logistic regression model P is a probability of having a cardiac condition. This probability would indicate a risk of having a cardiac condition and will be used by the clinical provider to direct the OSA patient to a cardiologist specialist for further investigation of the possible cardiac conditions.

According to an exemplary embodiment of the invention, the output of the model will be classified to high, medium and low risk similarly to the first model described by using thresholds defined by the user. Based on this output an alert could be raised if the score is sufficiently high on any given day. Alternatively, an averaged score over the last days will be used. Again, the user is free to choose on how many of the latest days they would like to calculate a risk average.

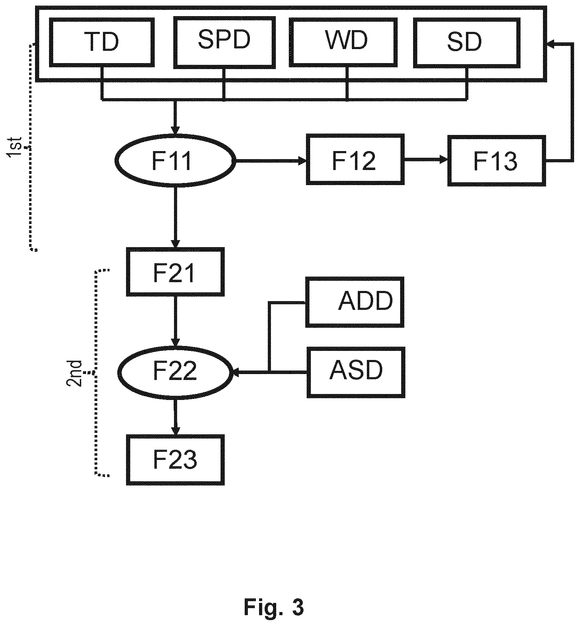

Fig. 3 shows a flow chart of a two-level algorithm or a two-level procedure for screening cardiac conditions of a patient according to an embodiment.

According to an exemplary embodiment of the invention, the therapy data TD, SpO2 data SPD, wearable data WD or smartphone data SD may be used as input data or therapy sensor data for the first level of a multi-level procedure, i.e. the therapy sensor data is provided from a first data source as sensor data during a therapy of the patient.

According to an exemplary embodiment of the invention, during the first level of a multi-level procedure, preferably a two-level procedure, in step F11 the risk of HF or cardiac condition may be determined, if the determined value is below a predefined threshold.

According to an exemplary embodiment of the invention, when performing step F12, a subgroup of patients with medium cardiac or HF risk may be determined. According to an exemplary embodiment of the invention, by executing step F13, a waiting time in terms of days, weeks or months for a newly initiated re-assessing of the risk may be awaited.

According to an exemplary embodiment of the invention, when performing step F21, the second level may start with continuing for the subgroup of patients with high cardiac risk or HF risk as determined in F11.

According to an exemplary embodiment of the invention, when performing step F22 the assessment certainty by refining may be achieved using additional device data add, or additional survey data ASD.

According to an exemplary embodiment of the invention, when performing step F23, the refined calculated risk may be provided.

Further, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It is noted that embodiments of the invention are described with reference to different subject matters. In particular, some embodiments are described with reference to method type claims whereas other embodiments are described with reference to the device type claims.

However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and the foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. A method for screening cardiac conditions of a patient, the method comprising the following steps:

    calculating (S1) a cardiac risk value based on therapy sensor data by means of a first level of a multi-level procedure, wherein the therapy sensor data is provided from a first data source as sensor data during a therapy of the patient; and
    refining (S2) the calculated cardiac risk value based on survey data and/or device data by means of a second level of a multi-level procedure to provide a refined cardiac risk value, wherein the survey data and/or the device data is provided from a second data source by incremental data gathering.

2. The method of claim 1, wherein the therapy sensor data comprises sensor data on ventilation pressure, ventilation volume, ventilation airflow rate, or oxygen partial pressure provided during respiratory ventilation as the therapy of the patient.

3. The method of claim 1 or 2, wherein the therapy sensor data comprises sensor data on a breath rate of the patient or respiration characteristics of the patient, the sensor data provided by at least one microphone recording the sound of breathing of the patient.

4. The method of any one of the preceding claims, wherein the survey data comprises data of a push survey of the patient.

5. The method of claim 4, wherein the push survey of the patent is conducted using an application program on a mobile electronic equipment.

6. The method of any one of the preceding claims, wherein the device data comprises data on blood pressure and/or on body weight.

7. The method of claim 6, wherein the device data is provided using an application program on a mobile electronic equipment.

8. The method of any one of the preceding claims, wherein the method further comprises the steps of further refining the refined cardiac risk value by additional data of the first data source or the second data source or a third data source and by means of a n-th level of a multi-level procedure; wherein n is greater than 2.

9. A medical screening system (100) for screening cardiac conditions of a patient, the medical screening system (100) comprising:

    a first data source (110);
    a second data source (120);
    at least one processor (130), at least configured to calculate a cardiac risk value based on therapy sensor data using a first level of a two-level procedure, wherein the therapy sensor data is provided from the first data source (110) as sensor data during a therapy of the patient; wherein the at least one processor (130) is further configured to refine the calculated cardiac risk value based on survey data and/or device data using a second level of a two-level procedure to provide a refined cardiac risk value, wherein the survey data and/or the device data is provided from the second data source (120) by incremental data gathering.

10. The medical screening system of claim 9, wherein the first data source (110) is a sensor configured to measure sensor data on ventilation pressure, ventilation volume, ventilation airflow rate, or oxygen partial pressure provided during respiratory ventilation as the therapy of the patient.

11. The medical screening system of claim 9 or 10, wherein the first data source (110) comprises a microphone configured to record the sound of breathing of the patient and the first data source (110) is configured to determine a breath rate of the patient or respiration characteristics of the patient based on the recorded sound of the breathing of the patient.

12. The medical screening system of claim any one of the preceding claims 9 to 11, wherein the second data source (120) is implemented on a mobile electronic equipment and the second data source (120) is configured to provide the survey data comprising data of a push survey provided by a survey of the patient, wherein the survey of the patent is conducted using an application program on a mobile electronic equipment.

13. A computer program element, which when executed by a processor is configured to carry out the method of any one of claims 1 to 8, and/or to control a medical screening system of claims 9 to 12.

14. A data carrier signal comprising therapy sensor data used by the method of any one of claims 1 to 8.

15. A data carrier signal comprising survey data and/or device data used by the method of any one of claims 1 to 8.

**Fig. 1**

**Fig. 2**

Fig. 3

Europäisches Patentamt

European Patent Office

Office européen des brevets

# EUROPEAN SEARCH REPORT

Application Number

EP 22 15 2851

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/353166 A1 (SIRENDI MAREK [EE] ET AL) 18 November 2021 (2021-11-18) | 1,8,9, 13-15 | INV. A61B5/0205 |
| Y | * the whole document * | 2-7, 10-12 | A61B5/00 |
| | ----- | | |
| X | US 2015/250428 A1 (ZHANG YI [US] ET AL) 10 September 2015 (2015-09-10) | 14,15 | |
| Y | * paragraph [0010] - paragraph [0032] * <br> * paragraph [0052] - paragraph [0088] * <br> * figures 1-7 * <br> * paragraph [0041] - paragraph [0045] * | 2,3,6,7, 10,11 | |
| | ----- | | |
| Y | US 2008/157980 A1 (SACHANANDANI HARESH G [US] ET AL) 3 July 2008 (2008-07-03) <br> * paragraph [0066] - paragraph [0089] * <br> * figures 1-10 * | 4,5,12 | |
| | ----- | | |
| A | US 2015/342487 A1 (THAKUR PRAMODSINGH HIRASINGH [US] ET AL) 3 December 2015 (2015-12-03) <br> * paragraph [0006] - paragraph [0058] * <br> * figures 1-7 * | 1-15 | |
| | ----- | | TECHNICAL FIELDS SEARCHED (IPC) |
| A | US 2003/149453 A1 (KROLL MARK W [US] ET AL) 7 August 2003 (2003-08-07) <br> * paragraph [0015] - paragraph [0020] * <br> * paragraph [0058] - paragraph [0075] * <br> * figures 1-4 * | 1-15 | A61B |
| | ----- | | |

The present search report has been drawn up for all claims

| Place of search | Date of completion of the search | Examiner |
|---|---|---|
| Berlin | 15 June 2022 | Abraham, Volkhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 22 15 2851**

This annex lists the patent family members relating to the patent documents cited in the above-mentioned European search report.
The members are as contained in the European Patent Office EDP file on
The European Patent Office is in no way liable for these particulars which are merely given for the purpose of information.

**15-06-2022**

| Patent document cited in search report | | Publication date | Patent family member(s) | | Publication date |
|---|---|---|---|---|---|
| US 2021353166 | A1 | 18-11-2021 | EP | 3846685 A1 | 14-07-2021 |
| | | | GB | 2582124 A | 16-09-2020 |
| | | | US | 2021353166 A1 | 18-11-2021 |
| | | | WO | 2020049267 A1 | 12-03-2020 |
| US 2015250428 | A1 | 10-09-2015 | CN | 106132286 A | 16-11-2016 |
| | | | EP | 3113674 A1 | 11-01-2017 |
| | | | US | 2015250428 A1 | 10-09-2015 |
| | | | US | 2017215809 A1 | 03-08-2017 |
| | | | WO | 2015134556 A1 | 11-09-2015 |
| US 2008157980 | A1 | 03-07-2008 | AU | 2007342524 A1 | 17-07-2008 |
| | | | EP | 2096995 A1 | 09-09-2009 |
| | | | EP | 2505132 A1 | 03-10-2012 |
| | | | JP | 5422392 B2 | 19-02-2014 |
| | | | JP | 2010514498 A | 06-05-2010 |
| | | | US | 2008157980 A1 | 03-07-2008 |
| | | | US | 2010045467 A1 | 25-02-2010 |
| | | | US | 2011319726 A1 | 29-12-2011 |
| | | | US | 2012271183 A1 | 25-10-2012 |
| | | | US | 2013245466 A1 | 19-09-2013 |
| | | | WO | 2008085309 A1 | 17-07-2008 |
| US 2015342487 | A1 | 03-12-2015 | CN | 106456003 A | 22-02-2017 |
| | | | EP | 3148422 A1 | 05-04-2017 |
| | | | US | 2015342487 A1 | 03-12-2015 |
| | | | WO | 2015187424 A1 | 10-12-2015 |
| US 2003149453 | A1 | 07-08-2003 | NONE | | |

EPO FORM P0459

For more details about this annex : see Official Journal of the European Patent Office, No. 12/82